(19) Europäisches Patentamt
European Patent Office
Office européen des brevets

(11) Publication number : **0 400 448 B1**

(12) **EUROPEAN PATENT SPECIFICATION**

(45) Date of publication of patent specification : **27.01.93 Bulletin 93/04**

(51) Int. Cl.$^5$ : **B01J 27/04,** C07C 2/84

(21) Application number : **90109639.6**

(22) Date of filing : **21.05.90**

(54) **Mixed basic metal sulfide catalyst.**

(30) Priority : **31.05.89 US 359207**
**31.05.89 US 359500**
**07.05.90 US 517068**

(43) Date of publication of application : **05.12.90 Bulletin 90/49**

(45) Publication of the grant of the patent : **27.01.93 Bulletin 93/04**

(84) Designated Contracting States : **DE FR GB IT NL**

(56) References cited :
**EP-A- 0 104 507**
**EP-A- 0 177 327**
**DE-A- 3 131 979**
**US-A- 4 544 785**
**US-A- 4 560 804**
**US-A- 4 571 290**

(73) Proprietor : **Institute of Gas Technology**
**3424 South State Street**
**Chicago, Illinois 60616 (US)**

(72) Inventor : **Erekson, Erek J.**
**22 S. Seventh Avenue**
**La Grange, Illin. 60525 (US)**
Inventor : **Lee, Anthony L.**
**305 Oak Street, Glen Ellyn**
**Illin. 60137 (US)**
Inventor : **Barone, Peter S.**
**1820 Sessions Walk, Hoffmam Estates,**
**Illinois (US)**
Inventor : **Solomon, Irvine J.**
**1005 Deerfield Place, Highland Park,**
**Illinois 60035 (US)**

(74) Representative : **Vogel, Georg**
**Pat.-Ing. Georg Vogel Hermann-Essig-Strasse 35 Postfach 11 65**
**W-7141 Schwieberdingen (DE)**

EP 0 400 448 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to mixed basic metal sulfide catalysts useful for production of higher hydrocarbons by oxidative coupling of aliphatic and alicyclic compounds, production of higher hydrocarbons by oxidative coupling of aliphatic and alicyclic hydrocarbon compounds with aliphatic and alicyclic substituted aromatic hydrocarbon compounds to form a longer substituent hydrocarbon on the aromatic ring, and production of unsaturated aliphatic and alicyclic chains by dehydrogenation of aliphatic and alicyclic hydrocarbon compounds and aliphatic and alicyclic substituted aromatic hydrocarbon compounds. Reaction of methane with oxygen in the presence of a mixed basic metal sulfide catalyst in accordance with this invention results in high conversion of methane with selectivity for ethane and ethylene products. Reaction of methane with toluene and oxygen in the presence of a mixed basic metal sulfide catalyst according to this invention results in high conversion to form styrene. One important dehydrogenation is the reaction of ethylbenzene in the presence of a mixed basic metal sulfide catalyst according to this invention to produce styrene.

Methane is currently available in large quantities from natural gas, anaerobic digestion of organic material, and chemical processing sources. However, use of methane as a chemical feedstock has been limited due to its high stability. It has been highly desirable to develop a catalyst for such reactions to enable operation under milder conditions with greater control over thermodynamic and kinetic processes as well as provide product selectivity and high reaction rate.

Oxidative coupling of methane to form higher hydrocarbons has been shown to be effected over a number of metal oxides, but yields of desired products have been low, as discussed by Keller, G.E. and M.M. Bhasin, J. of Catalysis 73, 9-19 (1982). Sodium and lead on alumina has been found to catalyze the formation of ethane and ethylene from methane, as disclosed in Hinsen, W. and M. Baerns, Chem.-Ztg., 107, 223-226 (1983) and Hinsen, W., W. Bytyn and M. Baerns, Proc. 8th Int. Congr. Catal., Berlin, III 581-592 (1984). Several U.S. patents teach a series of supported metal oxides which while effective for the conversion of methane to ethane and ethylene, are based on reducible metal oxides and used in a stoichiometric fashion by alternately exposing them to an oxidizing atmosphere and then to methane in the absence of oxygen. U.S. Patents 4,443,644; 4,443,645; 4,443,646; 4,443,647; 4,443,648; 4,443,649; 4,444,984, 4,499,322; 4,499,323; 4,499,324; and 4,523,049.

Later work has demonstrated that magnesium oxide and calcium oxide, when promoted with alkali metal salts, are active for oxidative coupling of methane to ethane and ethylene in the presence of oxygen. See Kimble, James B. and John H. Kolts, "Oxidative Coupling of Methane to Higher Hydrocarbons", Energy Progress, Vol. 6, p. 227 (1986); Driscoll, D.J., W.M. Martir, J. Wang and J.H. Lunsford, J. Am. Chem. Soc. 107, 58-63 (1985); and Ito, T., J. Wang, C. Lin and J.H. Lunsford, J. Am. Chem. Soc. 107, 5062-64 (1985). These later catalysts have the advantage of operating continuously, not requiring regeneration or pretreatment.

Borates and boron compounds have been used in partial oxidation of hydrocarbons, such as boric acid to oxidize long chain normal paraffins in the liquid phase (Illingworth, G.F. and G.W. Lester, ACS Petroleum Division Preprints, 12, No. 3, 161 (1967)) and oxidation of n-dodecane in the liquid phase to the corresponding alcohol (Lee, K.W., M.J. Choi, S.B. Kim and C.S. Choi, Ind. Eng. Chem. Res. 26, 1951 (1987)). Boric acid has been used by coating reactor walls in the combustion of methane to eliminate free radical destruction at temperatures of less than 513°C. (Kegeyan, E.M., I.S. Vardanyan and A.B. Nalbandyan, Kinetics and Catalysis 17, No. 4,749-754 and No. 4,755-759 (1976))

A number of publications describe oxidative methylation of toluene performed in Russia: Chemical Abstracts 97:127153K (1982) teaches non-catalytic methylation of toluene depended mostly on pressure and $PhMe/O/CH_4$ molar ratio; Chemical Abstracts 99:70137t (1983) teaches oxidative methylation of toluene using a Ni-V oxide or V oxide catalyst; Chemical Abstracts 101:74734t (1984) teaches oxidative methylation of toluene in presence of 0 (max. 15 percent in reaction mixture) results in products including styrene; Chemical Abstracts 101:38205 n (1984) teaches simultaneous production of styrene, ethylbenzene, benzene, and phenols by reaction of toluene with $C_{1-4}$ alkanes in the presence of O and $Fe_2O_3$ or $TiO_2$ at 600-800°. Productivity increased at higher pressure in presence of $H_2O_2$ and/or $(Me_3C)_2O_2$; and U.S. Patent 3,830,853 teaches reaction of toluene with a lower paraffin hydrocarbon in the presence of oxygen at 600°-900°C and space velocity of 2000-10000 $hour^{-1}$.

Styrene is an important commercial unsaturated aromatic monomer used extensively in the manufacture of plastics by polymerization and copolymerization. On a commercial scale, the great majority of the world's styrene is produced by dehydrogenation of ethylbenzene. A review of styrene synthesis processes is given in Kirk-Othmer, Encyclopedia of Chemical Technology, Third Edition, Vol. 21, Styrene, pgs. 770-801. One commercial process for production of styrene is the UOP Styro-Plus process using ethylbenzene and superheated steam under vacuum for the catalytic dehydrogenation of ethylbenzene as taught by Ward, D.J. et al, Hydrocarbon Processing, Vol. 66, No. 3, March 1987, pgs 47-48. Use of coke-covered alumina and boron/alumina catalysts for oxidative dehydrogenation of ethylbenzene is taught by Fiedorow, R., W. Przystajko, M. Sopa and I.G. Dalla Lana, The Nature and Catalytic Influence of Coke on Alumina: Oxidative Dehydrogenation of Ethylbenzene, Journal of Catalysis 68, pgs. 33-41 (1981). Oxidative dehydrogenation of ethylbenzene to styrene over metal pyrophosphates, such as cerium, tin, zirconium, and titanium phosphates and calcium magnesium, strontium, barium, nickel, aluminum, thorium, zinc and silicon phosphates is taught by Vrieland, G.E., Oxydehydration of Ethylbenzene to Styrene over Metal Phosphates, Journal of Catalysis 111, pgs. 1-13 (1988).This article teaches the condensed phosphate surface is the dominant factor as a catalyst and that the cation has little or no effect.

This invention provides a mixed basic metal sulfide catalyst and catalytic process for oxidative coupling of aliphatic and alicyclic compounds to produce higher molecular weight hydrocarbons and to dehydrogenate aliphatic and alicyclic hydrocarbons to form unsaturated compounds. The mixed basic metal sulfide catalyst of this invention provides sulfur tolerance which allows effective utilization of sulfur containing feedstocks as derived from naturally occurring carbonaceous materials.

The mixed basic metal sulfide catalyst of this invention has the formula:

xA.yB.zC.qS wherein

A is an alkali metal selected from lithium, sodium, potassium, rubidium, cesium and mixtures, thereof, preferably lithium;

B is selected from the group consisting of;

a cation which has an ionization state 1 greater than the ionization state of C wherein B is selected from scandium, yttrium, lanthanum, actinium, aluminum, boron, and mixtures thereof, preferably boron, aluminum yttrium, and lanthanum when C is selected from beryllium, magnesium, calcium, strontium, barium, radium, zinc, cadmium, mercury and mixtures thereof, preferably magnesium, calcium, barium and zinc; and B is selected from titanium, zirconium, hafnium, silicon and mixtures thereof, when C is selected from scandium, yttrium, lanthanum, actinium, aluminum, boron and mixtures thereof, preferably boron, aluminum, yttrium, and lanthanum; and

a cation which has an ionization state 2 and 3 greater than the ionization state of C wherein B is selected from hafnium, tantalum, niobium, vanadium and mixtures thereof, preferably tantalum; C is selected from magnesium, calcium, strontium, barium, and mixtures thereof, preferably magnesium;

x and y are in the mole fractions of z such that when z=1 then x=0.001 to 0.25, preferably 0.05 to 0.15 and y=0.001 to 0.25, preferably 0.002 to 0.20; and

q is a number necessary to maintain charge balance with S being sulfur.

The above mixed basic metal sulfide catalyst may be used in pure sulfide form or in any mixture with the mixed basic metal oxide catalyst set forth in European Patent Application 89103829.1-2104, filed March 4, 1989.

In a preferred embodiment, a boron/alkali metal promoted metal sulfide catalyst having boron in amounts of about 0.2 to about 20 mole percent (about 0.05 to about 5.0 weight percent), alkali metal promoter selected from the group consisting of lithium, sodium and potassium in amounts of about 0.1 to about 25 mole percent (about 0.1 to about 40 weight percent), metal sulfide selected from the group consisting of magnesium sulfide, calcium sulfide, zinc sulfide, and barium sulfide.

This invention provides a catalyst and process for oxidative coupling of aliphatic and alicyclic hydrocarbon compounds to produce a higher molecular weight hydrocarbon compound and for oxidative coupling of aliphatic and alicyclic hydrocarbon compounds with aliphatic and alicyclic substituted aromatic hydrocarbon compounds to produce a longer substituent hydrocarbon on the aromatic ring. The general reaction for oxidative coupling according to this invention may be expressed as:

$$R'H + R''H + 1/2O_2 \xrightarrow{cat} R'-R'' + H_2O$$

wherein R′H and R″H are each an aliphatic or alicyclic organic hydrocarbon compound which may be the same or different. For example, if both R′H and R″H were ethane, the reaction would be:

$$C_2H_6 + C_2H_6 + 1/2O_2 \xrightarrow{cat} C_4H_{10} + H_2O.$$

If R′H is propane and R″H is cyclohexane, a major reaction would be:

$$C_3H_8 + C_6H_{12} + 1/2O_2 \xrightarrow{cat} C_6H_{11}\text{-}C_3H_7 + H_2O$$

(1-propyl cyclohexane)

The catalyst does not prevent reaction of one compound with itself. Thus, when propane and cyclohexane are both fed to the reaction, propane may react with propane and cyclohexane may react with cyclohexane, but if desired, these reactions may be reduced by adjustment of the propane/cyclohexane feed ratio. In all instances of reaction according to this invention, oxidative coupling provides a process for producing higher molecular weight hydrocarbons. The reaction of an aliphatic or alicyclic hydrocarbon compound with an aliphatic or alicyclic substituted aromatic hydrocarbon compound and oxygen is conducted in the presence of a mixed basic metal sulfide catalyst at elevated temperature according to the following general reaction:

$$RH + R'CH_3 + O_2 \xrightarrow{cat} R\text{-}CH_2\text{-}R' + H_2O$$

wherein R is an aliphatic or alicyclic hydrocarbon radical;
and R'is an aliphatic or alicyclic hydrocarbon radical substituted on an aromatic hydrocarbon ring.

It is unexpected that catalysts active for oxidative coupling as described above involving carbon-carbon bond formation would also be active for dehydrogenation involving carbon-hydrogen bond breaking with subsequent carbon-carbon double bond formation.
Dehydrogenation of saturated organics has been described by Thomas, Charles L, Catalytic Processes and Proven Catalysts, Chap. 6, Dehydrogenation, pgs. 41-45, Academic Press (1970).

This invention provides a catalyst and process for dehydrogenation of aliphatic and alicyclic chains of aliphatic and alicyclic hydrocarbon compounds and aliphatic and alicyclic substituted aromatic hydrocarbon compounds to produce an unsaturation in the hydrocarbon chain. The reaction of an aliphatic or alicyclic hydrocarbon compound, an aliphatic or alicyclic substituted aromatic hydrocarbon compound and mixtures thereof in the dehydrogenation reaction is conducted in the presence of a mixed basic metal sulfide catalyst at elevated temperature. The dehydrogenation may proceed directly according to the following general reaction of C-C bonding in a compound RH or $R'CH_3$ being converted to C=C bonding + $H_2$ or may proceed by oxidative dehydrogenation wherein C-C bonding in a compound RH or $R'CH_3$ + 1/2 $O_2$ is converted to C=C bonding + $H_2O$, wherein R is an aliphatic or alicyclic hydrocarbon radical having 2 and more carbon atoms; and R' is an aliphatic or alicyclic hydrocarbon radical substituted on an aromatic hydrocarbon ring. In the case of dehydrogenation of ethylbenzene to styrene according to this invention, direct dehydrogenation proceeds according to the general reaction:

$$C_6H_5C_2H_5 \xrightarrow{cat} C_6H_5C_2H_3 + H_2$$

and by partial oxidation or oxidative dehydrogenation according to the general reaction:

$$C_6H_5C_2H_5 + 1/2O_2 \xrightarrow{cat} C_6H_5C_2H_3 + H_2O.$$

The mixed basic metal sulfide catalyst of this invention provides a catalyst which is tolerant of sulfur containing reactant compounds. Such sulfur tolerance is important when using hydrocarbon reactants derived from natural sources, such as methane obtained from gasification of coal, shale, and other natural carbonaceous materials. The maintenance of catalytic activity of the mixed basic metal sulfide catalyst of this invention in the presence of sulfur containing materials, such as $H_2S$, is of great commercial importance in view of the high cost of sulfur removal from hydrocarbons derived from naturally occurring sources.

The catalyst of this invention is a mixed basic metal sulfide catalyst having the formula xA.yB.zC.qS wherein A, B, C, x, y, z and q have the meanings set forth above with S being sulfur. In the catalyst formulation, B is a promoter on a C matrix to enhance active component A. The catalysts of this invention wherein the ionization state of B is 1 greater than C have only one oxidation state besides the metal, that is Ti, Zr, Hf and Si are only +4 and B, Al, Y and La are only +3, while Mg, Ca, Sr and Ba are only +2 and Li, K, Na, Rb and Cs are only +1. The catalysts of this invention wherein the ionization state of B is 2 and 3 greater than C the metal cations with an oxidation state besides the metal, of +4 and +5 modify a +2 oxidation state matrix to promote the desired

product selectivity.

In a particularly preferred embodiment, the catalyst of this invention is a boron/alkali metal promoted metal sulfide catalyst having boron in amounts of about 0.2 to about 20 mole percent (about 0.05 to about 5 weight percent) and preferably about 0.4 to about 2 mole percent (about 0.1 to about 0.5 weight percent); alkali metal promoter selected from the group consisting of lithium, sodium and potassium in amounts of about 0.1 to about 25 mole percent (about 0.1 to about 40 weight percent) and preferably about 0.5 to about 8 mole percent (about 0.5 to about 2.0 weight percent) and the remainder metal sulfide selected from the group consisting of magnesium sulfide, calcium sulfide, zinc sulfide, and barium sulfide. A preferred catalyst is boron/lithium promoted magnesium sulfide having about 0.20 to about 0.30 weight percent boron and about 0.8 to about 1.2 weight percent lithium.

The mixed basic metal sulfide catalyst of this invention may be used in its pure form or may be used in admixture with the mixed basic metal oxide catalyst described in the above copending commonly assigned patent application. When sulfur containing hydrocarbon feedstocks are used in the reactions catalyzed, it is preferred that the sulfide catalyst comprise over about 50 percent by weight of the total sulfide and oxide catalyst, and most preferably about 75 to about 100 percent.

The sulfide catalyst of this invention may be prepared by making a liquid solution of one or two soluble compounds of desired metal or metals or a colloidal suspension of solids in the liquid and adding it to a metal sulfide powder of the remaining component or components. Any liquids which will retain the sulfide compound are satisfactory. For example, an organic liquid must be used when using magnesium sulfide since an aqueous solution would cause undesired conversion to the oxide state. A wide variety of non-interfering ions may be used to form suitable liquid soluble compounds as long as they do not cause undesired chemical interference. Suitable such compounds include acids, sulfides, oxides, hydrides, and nitrates, carbonates, hydroxides. The liquid solution or colloidal suspension of one or two soluble components is added to metal sulfide powder of the remaining component or components and well mixed followed by drying at a sufficient temperature and for a sufficient time to expel volatile components. The mixture is then crushed and sieved to a small size for catalytic use. Conventional and well known catalyst manufacturing techniques may be employed to produce the catalyst material noted above. When preparing these catalytic materials, it is preferred to employ manufacturing techniques resulting in a product having a substantially uniform or homogeneous composition. Shaping of the material may be effected according to conventional techniques of the art, particularly tableting, or pelleting or extrusion. The catalyst may be used unsupported or alternatively it may be supported on an inert support as known to the art, such as alumina, silica, activated carbon and the like.

A 100 percent sulfide catalyst may be prepared by mixing 0.82 grams Cerac boron sulfide powder, -200 mesh, and 42.0 grams Cerac magnesium sulfide powder, -200 mesh, in a ceramic dish. 1.02 grams Aesar 99% lithium sulfide may be added to 30 grams n-propanol and stirred to obtain complete solution of the solids. The lithium solution is added to the boron and magnesium powders with stirring to obtain a homogeneous mixture which may then be dried at a temperature in excess of about 110°C. The dried mixture may then be calcined at a temperature of 700° to 750°C for a sufficient time, about 2 hours, to expel volatile portions. The mixture is then crushed and sieved to an appropriately small mesh size of about -6 to about +40, preferably about -12 to about +20 for use as a catalyst.

To prepare a mixed sulfide/oxide catalyst, a mixture of 0.43 grams Aesar 99.99% pure boric acid and 1.07 grams Aesar anhydrous lithium hydroxide and 30 grams n-propanol are added to a beaker and stirred to obtain complete solution of the solids. The solution is slowly added to 42.0 grams Cerac magnesium sulfide powder, -200 mesh, to obtain a homogeneous mixture which may be dried, calcined, and crushed.

The process of this invention provides a higher molecular weight hydrocarbon compound by gas phase oxidative coupling of saturated carbon atoms of one aliphatic or alicyclic hydrocarbon compound with a second aliphatic or alicyclic hydrocarbon compound and oxygen in the presence of a mixed basic metal sulfide catalyst as set forth above, such as a boron/alkali metal promoted metal sulfide catalyst.

Suitable aliphatic and alicyclic hydrocarbon compounds for use as feedstocks in the process of this invention include those compounds having up to eighteen carbon atoms. In the case of aliphatic compounds, $C_1$ through about $C_{18}$ are suitable; $C_1$ through about $C_{12}$ preferred; and $C_1$ through about $C_5$ most preferred. For alicyclic compounds $C_3$ through about $C_{18}$ are suitable and about $C_5$ through about $C_9$ preferred. The limiting factor for use as feedstocks in the process of this invention is that the material be gaseous at reaction conditions of temperature and pressure. Examplary preferred feedstocks include straight and branched chain saturated and unsaturated aliphatic hydrocarbons, such as methane, ethane, propane, butane, heptane, pentane, hexane, octane, isobutane, isohexane, isooctane, 1-pentene, 1-hexene and mixtures thereof; cyclic chain saturated and unsaturated alicyclic hydrocarbons, such as cyclobutane, cycloheptane, cycloheptene, cyclohexane, cyclohexene and mixtures thereof; and aryl substituted aliphatic and alicyclic hydrocarbons, such as toluene, xylene, mesitylene, durene, cumene and mixtures thereof. The aliphatic and alicyclic hydrocarbon compounds

used for oxidative coupling may be the same or different compounds. In the case of unsaturated hydrocarbons, it should be noted that the oxidative coupling of this invention does not occur at the unsaturated bonding. Feedstock gas comprising lower alkanes, principally methane, suitable for use in the process of this invention may comprise any methane containing gas which does not contain interfering compounds. Preferably, methane containing gas when used in the process of this invention comprises about 25 mole percent up to about 100 mole percent methane. Suitable sources of methane containing gas include natural gas, synthetic natural gas (SNG), product gas from gasification of carbonaceous materials, such as gasification of coal, peat, shale, and the like, as well as products of anaerobic digestion of various biomass materials. These gases principally comprise methane and may contain other hydrocarbon gases such as ethane and propane which may produce corresponding chemical reactions to those of methane in the process of this invention. Purification of such mixed gases comprising principally methane is not usually necessary, expecially when using the sulfide catalyst which has greater sulfur tolerance. These sources of methane containing gas and processes for producing methane are well known in the art.

Any oxygen containing gas not containing interfering chemical compounds is useful as a feedstock in oxidative coupling according to this invention. The term "oxygen containing gas" as used throughout this disclosure and claims, refers to gas containing oxygen, such as air and gases having an oxygen content of up to 100 percent. It is preferred to use oxygen containing gas comprising over 50 volume percent oxygen. The mole percentage of oxygen relative to the mole percentage of methane in the gas mixture subjected to the process of this invention is about 2 to about 40 and preferably about 5 to about 20 mole percent oxygen.

The reactants are fed to the reaction zone in mole percent proportions of about 80 to about 98 mole percent aliphatic or alicyclic hydrocarbon compounds, preferably about 88 to about 95 mole percent and about 2 to about 20 mole percent oxygen, preferably about 5 to about 12 mole percent. When different aliphatic and alicyclic compounds are used as reactants, they may be used in equal mole proportions or may be used in proportions of about 30 to about 70 mole percent one reactant to further desired reaction. Steam may be added in an amount of up to about 1 mole of steam per mole hydrocarbon to inhibit deep oxidation. Steam does not enter into the reaction but solely acts as an oxidation inhibitor.

The catalyst may be placed into a reactor, such as a tube-shell fixed bed, fluidized bed, moving bed, interbed heat exchange type, Fischer-Tropsch type, or other reactor type known to the art. Suitable reactor vessels for use at the desired operating temperatures and pressures are well known to the art. The reaction of methane and oxygen according to this invention is carried out by passing a gaseous mixture comprising methane and oxygen over the mixed basic metal sulfide catalyst as defined above at about 500° to about 1100°C, preferably about 600° to about 900°C. Suitable gas residence times are about 0.002 to about 0.00002 hour preferably about 0.0005 to about 0.0001 hour with space velocity of about 500 to about 50,000 vol/vol/hr, preferably about 1000 to about 5000 vol/vol/hr. The reaction may be carried out at about pressures of about 4.8kPa 1 psia) to about 7.25MPa (1515 psia), preferably about 0 to about 0.72MPa (150 psia). Suitable reactor vessels for use at the above operating temperatures and pressures are well known to the art. The products of the single reactor used in the process of this invention may be passed to a simple separator for separation of the hydrocarbon product, condensate, and vent gas.

The catalyst of this invention may be used in a process to provide a longer hydrocarbon substituent on an aromatic ring by gas phase oxidative coupling of saturated carbon atoms of an aliphatic or alicyclic hydrocarbon compound with an aliphatic or alicyclic substituted aromatic hydrocarbon and oxygen. Suitable aliphatic and alicyclic hydrocarbon compounds for use as feedstocks in the process of this invention include straight and branched chain saturated and unsaturated aliphatic hydrocarbons, such as methane, ethane, propane, butane, heptane, pentane, hexane, octane, isobutane, isohexane, isooctane, 1-pentene, 1-hexene and mixtures thereof; cyclic chain saturated and unsaturated alicyclic hydrocarbons, such as cyclobutane, cycloheptane, cycloheptene, cyclohexane, cyclohexene and mixtures thereof; and aryl substituted aliphatic and alicyclic hydrocarbons, such as toluene, xylene, mesitylene, durene, cumene and mixtures thereof. Suitable aliphatic and alicyclic substituted aromatic hydrocarbon compounds for use as feedstocks in this invention are aromatic ring hydrocarbons having at least one aliphatic or alicyclic hydrocarbon radical substituent on the aromatic ring, such as toluene, xylene, indan, tetralin, and mixtures thereof.

The reactants are fed to the reaction zone in mole percent proportions of about 50 to about 90 mole percent aliphatic or alicyclic hydrocarbon compounds, preferably about 75 to about 85 mole percent; about 2 to about 40 mole percent substituted aromatic hydrocarbon, preferably about 5 to about 15 mole percent; and about 2 to about 20 mole percent oxygen, preferably about 5 to about 12 mole percent. Steam may be added in an amount of up to about 1 mole of steam per mole hydrocarbon to inhibit deep oxidation. Steam does not enter into the reaction but solely acts as an oxidation inhibitor. It is preferred to use oxygen containing gas comprising over 50 volume percent oxygen. The amounts of oxygen used in the oxidative coupling of aliphatic and alicyclic hydrocarbons with aromatic hydrocarbons are expressed as pure oxygen. The oxygen containing gas may be

preheated by thermal exchange with the catalyst bed to a temperature suitable for the reaction controlling step of the process. An important aliphatic feedstock suitable for use in the process of this invention may comprise methane as described above. Important substituted aromatic feedstocks include toluene and xylene available from commercial sources.

The oxidative coupling is carried out by passing the gaseous aliphatic or alicyclic hydrocarbon and aromatic feedstocks and oxygen over the mixed basic metal sulfide catalyst as defined above under the reaction conditions of temperature, space velocity, and pressure set forth above for oxidative coupling of aliphatic and alicyclic compounds.

One important oxidative coupling reaction according to the process of this invention is the production of styrene directly by coupling of toluene and methane by the following reaction in the presence of the above defined catalyst:

$$C_6H_5CH_3 + CH_4 + O_2 \xrightarrow{cat} C_6H_5C_2H_3 + 2H_2O$$

At 750°C the heat of reaction (ΔH) is -73 kcal/mole and the sensible heat plus the heat of vaporization of toluene is about 55 kcal/mole. Thus the process operates close to autothermal conditions after initial light-off. Conventional processes using $Fe_2O_3$ as a catalyst with $Cr_2O_3$ as a stabilizer and $K_2CO_3$ as a coke retardant for production of styrene require ethylbenzene feedstock, produced from expensive benzene and ethylene and require large amounts of superheated steam (800°C and molar ratio 14 steam to 1 ethylbenzene) due to the conversion of ethylbenzene to styrene being endothermic. The process of this invention uses relatively inexpensive toluene, methane and air as feedstock to a single reactor where both styrene and ethylbenzene are produced in a process that does not require superheated steam.

The catalyst of this invention provides unsaturated aliphatic and alicyclic chains by dehydrogenation of saturated carbon atoms of an aliphatic or alicyclic hydrocarbon compound and an aliphatic or alicyclic substituted aromatic hydrocarbon and mixtures thereof. Suitable aliphatic and alicyclic hydrocarbon compounds for use as feedstocks in the process of this invention include straight and branched chain saturated aliphatic hydrocarbons, such as ethane, propane, butane, heptane, pentane, hexane, octane, isobutane, isohexane, isooctane and mixtures thereof; cyclic chain saturated alicyclic hydrocarbons, such as cyclobutane, cycloheptane, cyclohexane and mixtures thereof. Suitable aliphatic and alicyclic substituted aromatic hydrocarbon compounds for use as feedstocks in this invention are aromatic ring hydrocarbons having at least one saturated aliphatic or alicyclic hydrocarbon radical substituent on the aromatic ring, such as ethylbenzene, indan, tetralin and mixtures thereof.

The hydrocarbon reactant is fed to the reaction zone in contact with the above defined catalyst for direct dehydrogenation and for oxidative dehydrogenation. For oxidative dehydrogenation oxygen may be added up to a mole amount of about 5 moles oxygen per mole hydrocarbon, preferably about 0.5 to about 2.0 moles oxygen per mole hydrocarbon. Steam may be added in an amount of up to about 1 mole of steam per mole hydrocarbon to inhibit undesired side reactions when oxygen is used in the feed for oxidative dehydrogenation. Steam does not enter into the reaction but solely acts as an oxidation inhibitor. For direct dehydrogenation, without oxygen in the feed, steam may be used as a heat carrying agent and up to 10 moles of steam per mole of hydrocarbon may be required.

The dehydrogenation process according to this invention is carried out by passing the gaseous aliphatic or alicyclic hydrocarbon or aromatic feedstock over the mixed basic metal sulfide catalyst as defined above at a space velocity of about 500 to about 50,000 vol/vol/hr providing gas residence times of about 0.002 to about 0.00002 hour preferably about 0.0002 to about 0.00007 hour. Suitable temperatures are about 200° to about 1000°C, preferably about 600° to about 850°C for direct dehydrogenation and preferably about 450° to about 700°C for oxidative dehydrogenation. The reaction may be carried out at pressures of about 4.8kPa (1 psia) to about 7.25MPa (1515 psia), preferably about 4.8kPa (1 psia) to about 0.12MPa (25 psia) for direct dehydrogenation and preferably about 4.8kPa (1 psia) to about 0.72MPa (150 psia) for oxidative dehydrogenation. Pressures above atmospheric may enhance the rate of reaction. Suitable reactor vessels for use at the above operating temperatures and pressures are well known to the art. The products of the single reactor used in the process of this invention may be passed to a simple separator for separation of the hydrocarbon product, condensate, and vent gas.

One important dehydrogenation reaction according to the process of this invention is the production of styrene directly by dehydrogenation of ethylbenzene or by oxidative dehydrogenation of ethylbenzene in the presence of the above defined catalyst according to the reactions set forth above. At 727°C the heat of reaction (ΔH) for oxidative dehydrogenation is -29.4 kcal/mole exothermic and the sensible heat plus the heat of vaporization of ethylbenzene is about 33.0 kcal/mole. Thus the oxidative dehydrogenation process operates close to autothermal conditions after initial light-off. Conventional processes for production of styrene from ethylbenzene feedstock require large amounts of superheated steam (800°C and molar ratio 14 steam to 1 ethylbenzene) because the conversion of ethylbenzene to styrene is endothermic.The dehydration process of this invention uses a single reactor in a process that does not require superheated steam.

## Claims

1. A mixed basic metal sulfide catalyst having the formula:

   xA.yB.zC.qS wherein

   A is an alkali metal selected from lithium, sodium, potassium, rubidium, cesium and mixtures, thereof, preferably lithium;

   B is selected from the group consisting of;

   a cation which has an ionization state 1 greater than the ionization state of C wherein B is selected from scandium, yttrium, lanthanum, actinium, aluminum, boron, and mixtures thereof, preferably boron, aluminum yttrium, and lanthanum when C is selected from beryllium, magnesium, calcium, strontium, barium, radium, zinc, cadmium, mercury and mixtures thereof, preferably magnesium, calcium, barium and zinc; and B is selected from titanium, zirconium, hafnium, silicon and mixtures thereof, when C is selected from scandium, yttrium, lanthanum, actinium, aluminum, boron and mixtures thereof, preferably boron, aluminum, yttrium, and lanthanum; and

   a cation which has an ionization state 2 and 3 greater than the ionization state of C wherein B is selected from hafnium, tantalum, niobium, vanadium and mixtures thereof, preferably tantalum; C is selected from magnesium, calcium, strontium, barium, and mixtures thereof, preferably magnesium;

   x and y are in the mole fractions of z such that when z=1 then x=0.001 to 0.25, preferably 0.05 to 0.15 and y=0.001 to 0.25, preferably 0.002 to 0.20; and

   q is a number necessary to maintain charge balance with S being sulfur.

2. A catalyst according to Claim 1 wherein x=0.05 to 0.15 and y=0.002 to 0.20.

3. A catalyst according to Claim 1 wherein A is lithium.

4. A catalyst according to Claim 1 wherein B has an ionization state 1 greater than the ionization state of C and is selected from the group consisting of boron, aluminum, yttrium, lanthanum, and mixtures thereof and C is selected from the group consisting of magnesium, calcium, barium, zinc and mixtures thereof.

5. A catalyst according to Claim 1 wherein B has an ionization state 1 greater than the ionization state of C and is selected from the group consisting of silicon, titanium, zirconium, hafnium, and mixtures therof and C is selected from the group consisting of boron, aluminum, yttrium, lanthanum, and mixtures thereof.

6. A catalyst according to Claim 1 wherein B has an ionization state 2 and 3 greater than C.

7. A catalyst according to Claim 6 wherein B is tantalum and C is magnesium.

8. A process for producing higher molecular weight hydrocarbons by gas phase oxidative coupling of saturated carbon atoms of one aliphatic or alicyclic hydrocarbon compound with a second aliphatic of alicyclic hydrocarbon compound, said process comprising:

   oxidative coupling compounds selected from aliphatic hydrocarbon compounds, alicyclic hydrocarbon compounds, and mixtures thereof having up to 18 carbon atoms each to form a higher molecular weight hydrocarbon compound in the presence of oxygen and a mixed basic metal catalyst having the formula:

   xA.yB.zC.qS wherein

   A is an alkali metal selected from lithium, sodium, potassium, rubidium, cesium and mixtures, thereof, preferably lithium:

   B is selected from the group consisting of;

   a cation which has an ionization state 1 greater than the ionization state of c wherein B is selected from scandium, yttrium, lanthanum, actinium, aluminum, boron, and mixtures thereof, preferably boron, aluminum yttrium, and lanthanum when C is selected from beryllium, magnesium, calcium, strontium, barium, radium, zinc, cadmium, mercury and mixtures thereof, preferably magnesium, calcium, barium and zinc; and B is selected from titanium, zirconium, hafnium, silicon and mixtures thereof, when C is selected from scandium, yttrium, lanthanum, actinium, aluminum, boron and mixtures thereof, preferably

boron, aluminum, yttrium, and lanthanum; and

a cation which has an ionization state 2 and 3 greater than the ionization state of C wherein B is selected from hafnium, tantalum, niobium, vanadium and mixtures thereof, preferably tantalum; C is selected from magnesium, calcium, strontium, barium, and mixtures thereof, preferably magnesium;

x and y are in the mole fractions of z such that when z=1 then x=0.001 to 0.25, preferably 0.05 to 0.15 and y=0.001 to 0.25, preferably 0.002 to 0.20; and

q is a number necessary to maintain charge balance with S being sulfur.

9. A process according to Claim 8 wherein said aliphatic and alicyclic hydrocarbon compounds are selected from straight and branched chain saturated and unsaturated aliphatic hydrocarbons, cyclic chain saturated and unsaturated alicyclic hydrocarbons, and aryl substituted aliphatic and alicyclic hydrocarbons wherein said aliphatic compounds have 1 to about 12 and said alicyclic compounds have about 5 to about 9 carbon atoms.

10. A process according to Claim 8 wherein said aliphatic hydrocarbon compounds are selected from methane, ethane, propane, butane, heptane, pentane, hexane, octane, isobutane, isohexane, isooctane, 1-pentene, 1-hexene and mixtures thereof.

11. A process according to Claim 8 wherein said alicyclic hydrocarbon compounds are selected from cyclobutane, cycloheptane, cycloheptene, cyclohexane, cyclohexene, and mixtures thereof.

12. A process according to Claim 9 wherein said aryl substituted aliphatic and alicyclic hydrocarbon compounds are selected from toluene, xylene,mesitylene, durene, cumene and mixtures thereof.

13. A catalyst according to Claim 10 wherein one of said aliphatic hydrocarbon compounds is methane.

14. A process for producing higher molecular weight hydrocarbons by forming longer substituent hydrocarbon on an aromatic ring, said process comprising:

oxidative coupling a compound selected from aliphatic hydrocarbon compounds, alicyclic hydrocarbon compounds, and mixtures thereof with a compound selected from aliphatic substituted aromatic hydrocarbon compounds, alicyclic substituted aromatic hydrocarbon compounds, and mixtures thereof in the presence of oxygen and a mixed basic metal sulfide catalyst having the formula:

xA.yB.zC.qS wherein

A is an alkali metal selected from lithium, sodium, potassium, rubidium, cesium and mixtures, thereof, preferably lithium;

B is selected from the group consisting of;

a cation which has an ionization state 1 greater than the ionization state of C wherein B is selected from scandium, yttrium, lanthanum, actinium, aluminum, boron, and mixtures thereof, preferably boron, aluminum yttrium, and lanthanum when C is selected from beryllium, magnesium, calcium, strontium, barium, radium, zinc, cadmium, mercury and mixtures thereof, preferably magnesium, calcium, barium and zinc; and B is selected from titanium, zirconium, hafnium, silicon and mixtures thereof, when C is selected from scandium, yttrium, lanthanum, actinium, aluminum, boron and mixtures thereof, preferably boron, aluminum, yttrium, and lanthanum; and

a cation which has an ionization state 2 and 3 greater than the ionization state of C wherein B is selected from hafnium, tantalum, niobium, vanadium and mixtures thereof, preferably tantalum; C is selected from magnesium, calcium, strontium, barium, and mixtures thereof, preferably magnesium;

x and y are in the mole fractions of z such that when z=1 then x=0.001 to 0.25, preferably 0.05 to 0.15 and y=0.001 to 0.25, preferably 0.002 to 0.20; and

q is a number necessary to maintain charge balance with S being sulfur.

15. A process according to Claim 14 wherein said aliphatic hydrocarbon compound is methane and said aromatic hydrocarbon compound is toluene.

16. A process for producing unsaturated aliphatic and alicyclic hydrocarbon chains by dehydrogenation, said process comprising:

dehydrogenating a compound selected from aliphatic hydrocarbon compounds, alicyclic hydrocarbon compounds, aliphatic substituted aromatic hydrocarbon compounds, alicyclic substituted aromatic hydrocarbon compounds, and mixtures thereof in the presence of a mixed basic metal sulfide catalyst having the formula:

xA.yB.zC.qS wherein

A is an alkali metal selected from lithium, sodium, potassium, rubidium, cesium and mixtures, thereof, preferably lithium;

B is selected from the group consisting of;

a cation which has an ionization state 1 greater than the ionization state of C wherein B is selected from scandium, yttrium, lanthanum, actinium, aluminum, boron, and mixtures thereof, preferably boron, aluminum yttrium, and lanthanum when C is selected from beryllium, magnesium, calcium, strontium, barium, radium, zinc, cadmium, mercury and mixtures thereof, preferably magnesium, calcium, barium and zinc; and B is selected from titanium, zirconium, hafnium, silicon and mixtures thereof, when C is selected from scandium, yttrium, lanthanum, actinium, aluminum, boron and mixtures thereof, preferably boron, aluminum, yttrium, and lanthanum; and

a cation which has an ionization state 2 and 3 greater than the ionization state of C wherein B is selected from hafnium, tantalum, niobium, vanadium and mixtures thereof, preferably tantalum; C is selected from magnesium, calcium, strontium, barium, and mixtures thereof, preferably magnesium;

x and y are in the mole fractions of z such that when z=1 then x=0.001 to 0.25, preferably 0.05 to 0.15 and y=0.001 to 0.25, preferably 0.002 to 0.20; and

q is a number necessary to maintain charge balance with S being sulfur.

**17.** A process according to Claim 16 wherein said hydrocarbon compound is ethylbenzene.

## Patentansprüche

**1.** Gemischter basischer Metallsulfid-Katalysator mit der Formel: xA.yB.zC.qS wobei
A ein Alkalimetall ist, das aus Lithium, Natrium, Kalium, Rubidium, Zäsium und Mischungen daraus ausgewählt ist, insbesondere Lithium;
B aus der Gruppe gewählt ist, bestehend aus:
einem Kation, das einen Ionisationszustand um 1 größer als der Ionisationszustand von C hat, worin B gewählt ist aus Scandium, Yttrium, Lanthan, Actinium, Aluminium, Bor und Mischungen daraus, vorzugsweise Bor, Aluminium Yttrium und Lanthan, wenn C gewählt ist aus Beryllium, Magnesium, Calcium, Strontium, Barium, Radium, Zink, Cadmium, Quecksilber und Mischungen daraus, vorzugsweise Magnesium, Calcium, Barium und Zink; und
B gewählt ist aus Titan, Zirkonium, Hafnium, Silicium und Mischungen daraus, wenn C gewählt ist aus Scandium, Yttrium, Lanthan, Actinium, Aluminium, Bor und Mischungen daraus, vorzugsweise Bor, Aluminium, Yttrium und Lanthan; und
einem Kation, das einen Ionisationszustand hat, der um 2 oder 3 größer ist als der Ionisationszustand von C, worin B ausgewählt ist aus Hafnium, Tantal, Niob, Vanadium und Mischungen daraus, vorzugsweise Tantal; C gewählt ist aus Magnesium, Calcium, Strontium, Barium und Mischungen daraus, vorzugsweise Magnesium;
x und y in Molanteilen von z ausgedrückt sind, derart, daß, wenn z = 1 ist, x = 0,001 bis 0,25 beträgt, vorzugsweise 0,05 bis 0,15, und y = 0,001 bis 0,25, vorzugsweise 0,002 bis 0.20 beträgt; und
q eine Zahl ist, die notwendig ist, um das Ladungsgleichgewicht mit S, das Schwefel bedeutet, aufrechtzuerhalten.

**2.** Katalysator nach Anspruch 1,
worin x = 0,05 bis 0,15 und y = 0,002 bis 0,20 ist.

**3.** Katalysator nach Anspruch 1,
worin A Lithium ist.

**4.** Katalysator nach Anspruch 1,
worin B einen Ionisationszustand um 1 größer als der Ionisationszustand von C hat und aus der Gruppe ausgewählt ist, bestehend aus Bor, Aluminium, Yttrium, Lanthan und Mischungen daraus und C aus der Gruppe ausgewählt ist, bestehend aus Magnesium, Calcium, Barium, Zink und Mischungen daraus.

**5.** Katalysator nach Anspruch 1,
worin B einen Ionisationszustand um 1 größer als der Ionisationszustand von C hat und ausgewählt ist aus der Gruppe, bestehend aus Silicium, Titan, Zirkonium, Hafnium und Mischungen daraus und C ausgewählt ist aus der Gruppe, bestehend aus Bor, Aluminium, Yttrium, Lanthan und Mischungen daraus.

6. Katalysator nach Anspruch 1,
worin B einen Ionisationszustand um 2 oder 3 größer als C hat.

7. Katalysator nach Anspruch 6,
worin B Tantal und C Magnesium ist.

8. Verfahren zum Herstellen von Kohlenwasserstoffen mit höherem Molekulargewicht durch oxydative Gasphasen-Kopplung gesättigter Kohlenstoffatome einer aliphatischen oder alizyklischen Kohlenwasserstoffverbindung mit einer zweiten aliphatischen oder alizyklischen Kohlenwasserstoffverbindung, wobei das Verfahren umfaßt:
Verbindungen zum oxydativen Koppeln ausgewählt aus aliphatischen Kohlenwasserstoffverbindungen, alizyklischen Kohlenwasserstoffverbindungen und Mischungen daraus mit bis zu 18 Kohlenstoffatomen, jeweils zum Bilden von Kohlenwasserstoffverbindungen mit höherem Molekulargewicht in Anwesenheit von Sauerstoff und einem gemischten basischen Metallkatalysator mit der Formel: xA.yB.zC.qS, worin A ein Alkalimetall ist, das ausgewählt ist aus Lithium, Natrium, Kalium, Rubidium, Zäsium und Mischungen daraus, vorzugsweise Lithium;
B ausgewählt ist aus der Gruppe bestehend aus:
einem Kation, das einen Ionisationszustand um 1 größer als der Ionisationszustand von C hat, worin B ausgewählt ist aus Scandium, Yttrium, Lanthan, Aktinium, Aluminium, Bor und Mischungen daraus, vorzugsweise Bor, Aluminium Yttrium und Lanthan, wenn C ausgewählt ist aus Beryllium, Magnesium, Kalzium, Strontium, Barium, Radium, Zink, Cadmium, Quecksilber und Mischungen daraus, vorzugsweise Magnesium, Calcium, Barium und Zink; und B ausgewählt ist aus Titan, Zirkonium, Hafnium, Silicium und Mischungen daraus, wenn C ausgewählt ist aus Scandium, Yttrium, Lanthan, Actinium, Aluminium, Bor und Mischungen daraus, vorzugsweise Bor, Aluminium, Yttrium und Lanthan; und
einem Kation, das einen Ionisationszustand um 2 oder 3 größer hat als der Ionisationszustand von C, worin B ausgewählt ist aus Hafnium, Tantal, Niob, Vanadium und Mischungen daraus, vorzugsweise Tantal; C ausgewählt ist aus Magnesium, Calcium, Strontium, Barium und Mischungen daraus, vorzugsweise Magnesium;
x und y in Molanteilen von z bestehen, derart, daß, wenn z = 1 ist, x = 0,001 bis 0,25, vorzugsweise 0,05 bis 0,15, und y = 0.001 bis 0,25, vorzugsweise 0,002 bis 0,2; und
q eine Zahl ist, die notwendig ist, um das Ladungsgleichgewicht mit S, das Schwefel bedeutet, aufrechtzuerhalten.

9. Verfahren nach Anspruch 8,
wobei die aliphatischen und alizyklischen Kohlenwasserstoffverbindungen ausgewählt sind aus geraden und verzweigten Ketten gesättigter und ungesättigter aliphatischer Kohlenwasserstoffe, zyklischer gesättigter und ungesättigter Ketten alizyklischer Kohlenwasserstoffe und Aryl substituierte aliphatische und alizyklische Kohlenwasserstoffe, wobei die aliphatischen Verbindungen 1 bis etwa 12 und die alizyklischen Verbindungen etwa 5 bis etwa 9 Kohlenstoffatome aufweisen.

10. Verfahren nach Anspruch 8,
wobei die aliphatischen Kohlenwasserstoffverbindungen ausgewählt sind aus Methan, Äthan, Propan, Butan, Heptan, Pentan, Hexan, Octan, Isobutan, Isohexan, Isooctan, 1-Penten, 1-Hexen und Mischungen daraus.

11. Verfahren nach Anspruch 8,
wobei die alizyklischen Kohlenwasserstoffverbindungen ausgewählt sind aus Cyclobutan, Cycloheptan, Cyclohepten, Cyclohexan, Cyclohexen und Mischungen daraus.

12. Verfahren nach Anspruch 9,
wobei die Aryl substituierten aliphatischen und alizyklischen Kohlenwasserstoffverbindungen ausgewählt sind aus Toluen, Xylen, Mesitylen, Duren, Cumol und Mischungen daraus.

13. Katalysator nach Anspruch 10,
wobei eine der aliphatischen Kohlenwasserstoffverbindungen Methan ist.

14. Verfahren zum Erzeugen von Kohlenwasserstoffen höheren Molekulargewichts durch Bilden eines längeren substituierten Kohlenwasserstoffes auf einem aromatischen Ring, wobei das Verfahren umfaßt: oxydative Kopplung einer Verbindung, ausgewählt aus aliphatischen Kohlenwasserstoffverbindungen, alizyklischen Kohlenwasserstoffverbindungen und Mischungen daraus mit einer Verbindung, ausgewählt aus aliphatischen substituierten aromatischen Kohlenwasserstoffverbindungen, alizyklischen substituierten aromatischen Kohlenwasserstoffverbindungen und Mischungen daraus in Anwesenheit von Sauerstoff und einem gemischten basischen Metallsulfid-Katalysator mit der Formel: xA.yB.zC.qS wobei A ein alkalisches Metall ist, ausgewählt aus Lithium, Natrium, Kalium, Rubidium, Zäsium und Mischungen daraus, vorzugsweise Lithium;
B ausgewählt ist aus der Gruppe bestehend aus:
einem Kation, das einen Ionisationszustand um 1 größer als der Ionisationszustand von C hat, wobei B ausgewählt ist aus Scandium, Yttrium, Lanthan, Actinium, Aluminium, Bor und Mischungen daraus, insbesondere Bor, Aluminium, Yttrium und Lanthan, wenn C ausgewählt ist aus Beryllium, Magnesium, Calcium, Strontium, Barium, Radium, Zink, Cadmium, Quecksilber und Mischungen daraus, vorzugsweise Magnesium, Calcium, Barium und Zink; und B ausgewählt ist aus Titan, Zirkonium, Hafnium, Silicium und Mischungen daraus, wenn C ausgewählt ist Scandium, Yttrium, Lanthan, Actinium, Aluminium, Bor und Mischungen daraus, insbesondere Bor, Aluminium, Yttrium, und Lanthan; und
einem Kation, das einen Ionisationszustand um 2 oder 3 größer hat als der Ionisationszustand von C, wobei B ausgewählt ist aus Hafnium, Tanthal, Niob, Vanadium und Mischungen daraus, vorzugsweise Tanthal; C ausgewählt ist aus Magnesium, Calcium, Strontium, Barium und Mischungen daraus, vorzugsweise Magnesium;
x und y Molanteile von z derart bilden, daß, wenn z = 1 ist, x = 0,001 bis 0,25 ist, vorzugsweise 0,05 bis 0,15, und y = 0,001 bis 0,25 ist, vorzugsweise 0,002 bis 0,20; und
q eine Zahl ist, die erforderlich ist, um das Gleichgewicht mit S, das Schwefel bezeichnet, aufrechtzuerhalten.

**15.** Verfahren nach Anspruch 14,
wobei die aliphatische Kohlenwasserstoffverbindung Methan ist und die aromatische Kohlenwasserstoffverbindung Toluol ist.

**16.** Verfahren zum Erzeugen ungesättigter aliphatischer und alizyklischer Kohlenwasserstoffketten durch Dehydration, wobei das Verfahren umfaßt:
Dehydrieren einer Verbindung ausgewählt aus aliphatischen Kohlenwasserstoffverbindungen, alizyklischen Kohlenwasserstoffverbindungen, aliphatischen substituierten aromatischen Kohlenwasserstoffverbindungen, alizyklischen substituierten aromatischen Kohlenwasserstoffverbindungen und Mischungen daraus in Anwesenheit eines gemischten basischen Metallsulfid-Katalysators mit der Formel:

xA.yB.zC.qS,

wobei A ein Alkalimetall ist, ausgewählt ist aus Lithium, Natrium, Kalium, Rubidium, Zäsium und Mischungen daraus, vorzugsweise Lithium;
B ausgewählt ist aus der Gruppe bestehend aus:
einem Kation, das einen Ionisationszustand um 1 größer als der Ionisationszustand von C hat, wobei B ausgewählt ist aus Scandium, Yttrium, Lanthan, Actinium, Aluminium, Bor oder Mischungen daraus, vorzugsweise Bor, Aluminium Yttrium und Lanthan, wenn C ausgewählt ist aus Beryllium, Magnesium, Calcium, Strontium, Barium, Radium, Zink, Cadmium, Quecksilber und Mischungen daraus, vorzugsweise Magnesium, Calcium, Barium und Zink; und B ausgewählt ist aus Titan, Zirkonium, Hafnium, Silicium und Mischungen daraus, wenn C ausgewählt ist aus Scandium, Yttrium, Lanthan, Actinium, Aluminium, Bor und Mischungen daraus, vorzugsweise Bor, Aluminium, Yttrium und Lanthan; und
einem Kation, das einen Ionisationszustand um 2 oder 3 größer hat als der Ionisationszustand von C, wobei B ausgewählt ist aus Hafnium, Tantal, Niob, Vanadium und Mischungen daraus, vorzugsweise Tantal; C ausgewählt ist aus Magnesium, Calcium, Strontium, Barium und Mischungen daraus, vorzugsweise Magnesium;
x und y Molanteile von z derart bilden, daß, wenn z =1 ist, x = 0,001 bis 0,25 ist, vorzugsweise 0,05 bis 0,15, und y = 0,001 bis 0,25 ist, vorzugsweise 0,002 bis 0,20; und
q eine Zahl ist, die notwendig ist, um das Gleichgewicht mit S, das Schwefel bezeichnet, aufrechtzuerhalten.

**17.** Verfahren nach Anspruch 16,
wobei die Kohlenwasserstoffverbindung Äthylbenzol ist.

## Revendications

**1.** Catalyseur basique constitué de sulfure métallique mixte représenté par la formule:

xA.yB.zC.qS dans laquelle

A est un métal alcalin choisi parmi le lithium, le sodium,le potassium, le rubidium , le césium et des mélanges de ceux-ci, avec une préférence pour le lithium;

B est choisi dans le groupe comprenant:

un cation qui a un degré d'ionisation une fois plus grand que le degré d'ionisation de C dans lequel B est choisi parmi le scandium, l'yttrium, le lanthane, l'actinium, l'aliminium, le bore et des mélange s de ceux-ci, avec une préférence pour le bore, l'aluminium, l'yttrium et le lanthane lorsque C est choisi parmi le béryllium, le magnésium, le calcium, le strontium, le baryum, le radium, le zinc, le cadmium, le mercure et des mélanges de ceux-ci, avec une préférence pour le magnésium, le calcium, le baryum et le zinc; et B est choisi parmi le titane, le zirconium, le hafnium, le silicium et des mélanges de ceux-ci, lorsque C est choisi parmi le scandium, l'yttrium, le lanthane, l'actinium, l'aluminium, le bore et des mélanges de ceux-ci, avec une préférence pour le bore, l'aluminium l'yttrium et le lanthane; et

un cation qui a un degré d'ionisation deux et trois fois plus grand que le degré d'isonisation de C dans lequel B est choisi parmi le hafnium, le tantale, le niobium, le vanadium et des mélanges de ceux-ci, avec une préférence pour le tantale; C est choisi parmi le magnésium, le calcium, le strontium, le baryum et des mélanges de ceux-ci, avec une préférence pour le magnésium;

x et y sont dans des rapports molaires à z tels que lorsque z=1, alors x=0,001 à 0,25 , et de préférence 0,05 à 0,15 et y=0,001 à 0,25, de préférence 0,002 à 0,20; et

q est le nombre nécessaire pour maintenir l'équilibre des charges avec S, qui est du soufre.

**2.** Catalyseur suivant la revendication 1, dans lequel x=0,05 à 0,15 et y=0,002 à 0,20.

**3.** Catalyseur suivant la revendication 1, dans lequel A est du lithium.

**4.** Catalyseur suivant la revendication 1 dans lequel B possède un degré d'iuonisation une fois plus grand que le degré d'ionisation de C et est choisi parmi le groupe constitué de silicium, de titane, d'yttrium, de lanthane, et des mélanges de ceux-ci, et C est choisi dans le groupe constitué de magnésium, de calcium, de baryum, de zinc et des mélanges de ceux-ci.

**5.** Catalyseur suivant la revendication 1 dans lequel B possède un degré d'ionisation une fois plus grand que le degré d'ionisation de C et est choisi parmi le groupe constitué de silicium, de titane, de zirconium, de hafnium, et des mélanges de ceux-ci et C est choisi parmi le groupe constitué de bore, d'aluminium, d'yttrium, de lanthane et des mélanges de ceux-ci.

**6.** Catalyseur suivant la revendication 1 dans lequel B possède un degré d'ionisation deux et trois fois plus grand que C.

**7.** Catalyseur suivant la revendication 6 dans lequel B est du tantale et C est du magnésium.

**8.** Procédé de production d'hydrocarbures de poids moléculaires plus élevés par condensation oxydative en phase gazeuse d'atomes de carbone saturés d'un composé hydrocarboné aliphatique ou alicyclique avec un second composé hydrocarboné aliphatique ou alicyclique , ledit procédé comprenant:

la condensation oxydative de composés choisi parmi des composés hydrocarbonés aliphatiques, des composés hydrocarbonés alicycliques et des mélanges de ceux-ci ayant jusqu'à 18 atomes de carbone chacun pour former des composés hydrocarbonés de poids moléculaires plus élevés en présence d'oxygène et d'un catalyseur métallique basique mixte ayant la formule:

xA.yB.zC.qS dans laquelle

A est un métal alcalin choisi parmi le lithium, le sodium,le potassium, le rubidium , le césium et des mélanges de ceux-ci, avec une préférence pour le lithium;

B est choisi dans le groupe comprenant:

un cation qui a un degré d'ionisation une fois plus grand que le degré d'ionisation de C dans lequel B est choisi parmi le scandium, l'yttrium, le lanthane, l'actinium, l'aliminium, le bore et des mélanges de ceux-ci, avec une préférence pour le bore, l'aluminium, l'yttrium et le lanthane lorsque C est choisi parmi le béryllium, le magnésium, le calcium, le strontium, le baryum, le radium, le zinc, le cadmium, le mercure et des mélanges de ceux-ci, avec une préférence pour le magnésium, le calcium, le baryum et le zinc; et B est choisi parmi le titane, le zirconium, le hafnium, le silicium et des mélanges de ceux-ci, lorsque C est

choisi parmi le scandium, l'yttrium, le lanthane, l'actinium, l'aluminium, le bore et des mélanges de ceux-ci, avec une'préférence pour le bore, l'aluminium l'yttrium et le lanthane; et

un cation qui a un degré d'ionisation deux et trois fois plus grand que le degré d'isonisation de C dans lequel B est choisi parmi le hafnium, le tantale, le niobium, le vanadium et des mélanges de ceux-ci, avec une préférence pour le tantale; C est choisi parmi le magnésium, le calcium, le strontium, le baryum et des mélanges de ceux-ci, avec une préférence pour le magnésium;

x et y sont dans des rapports molaires à z tels que lorsque z=1, alors x=0,001 à 0,25 , et de préférence 0,05 à 0,15 et y=0,001 à 0,25, de préférence 0,002 à 0,20; et

q est le nombre nécessaire pour maintenir l'équilibre des charges avec S, qui est du soufre.

**9.** Procédé suivant la revenduication 8, dans lequel lesdits composés hydrocarbonés aliphatiques et alicycliques sont choisis parmi le groupe comprenant des hydrocarbunes aliphatiques saturés et insaturés à chaîne droite ou ramifiée, des hydrocarbures alicycliques saturés et insaturés à chaîne cycliques et des hydrocarbures aliphatiques et alicycliques aryle substitués dans lesquels lesdits composés aliphatiques possèdent de 1 à environ 12 atomes de carbone et dans lesquels lesdits composés alicycliques possèdebt de 5 à environ 9 atomes de carbone.

**10.** Procédé suivant la revendication 8 dans lequel lesdits composés hydrocarbonés aliphatiques sont choisis parmi le groupe comprenant le méthane, l'éthane, ele propane, le butane, l'heptane, le pentane, l'hexane, l'octane, l'isobutane, l'isohexane, l'isooctane, le 1-pentène, le 1-hexène et des mélanges de ceux-ci.

**11.** Procédé suivant la revendication 8 dans lequel lesdits composés hydrocarbonés alicycliques sont choisis parmi le groupe comprenant le cyclobutane, le cycloheptane, le cycloheptane, le cyclohexane, le cyclohexène et des mélanges de ceux-ci.

**12.** Procédé suivant la revendication 9 dans lequel lesdits composés hydrocarbonés aliphatiques et alicycliques aryle substitués sont choisis parmi le groupe comprenant le toluène, le xylène, le mésitylène, le durène, le cumène et des mélanges de ceux-ci.

**13.** Catalyseur suivant la revendication 10 dans lequel l'un desdits composés hydrocarbonés aliphatiques est du méthane.

**14.** Procédé de production d'hydrocarbures à poids moléculaires plus élevés par la formation d'un hydrocarbure de substitution plus long sur un noyau aromatique, ledit procédé comprenant:

la condensation oxydative de composés choisi parmi des composés hydrocarbonés aliphatiques, des composés hydrocarbonés alicycliques et des mélanges de ceux-ci ayant jusqu'à 18 atomes de carbone chacun pour former des composés hydrocarbonés de poids moléculaires plus élevés en présence d'oxygène et d'un catalyseur basique constitué de sulfure métallique mixte ayant la formule:

xA.yB.zC.qS dans laquelle

A est un métal alcalin choisi parmi le lithium, le sodium,le potassium, le rubidium , le césium et des mélanges de ceux-ci, avec une préférence pour le lithium;

B est choisi dans le groupe comprenant:

un cation qui a un degré d'ionisation une fois plus grand que le degré d'ionisation de C dans lequel B est choisi parmi le scandium, l'yttrium, le lanthane, l'actinium, l'aliminium, le bore et des mélanges de ceux-ci, avec une préférence pour le bore, l'aluminium, l'yttrium et le lanthane lorsque C est choisi parmi le béryllium, le magnésium, le calcium, le strontium, le baryum, le radium, le zinc, le cadmium, le mercure et des mélanges de ceux-ci, avec une préférence pour le magnésium, le calcium, le baryum et le zinc; et B est choisi parmi le titane, le zirconium, le hafnium, le silicium et des mélanges de ceux-ci, lorsque C est choisi parmi le scandium, l'yttrium, le lanthane, l'actinium, l'aluminium, le bore et des mélanges de ceux-ci, avec une préférence pour le bore, l'aluminium l'yttrium et le lanthane; et

un cation qui a un degré d'ionisation deux et trois fois plus grand que le degré d'isonisation de C dans lequel B est choisi parmi le hafnium, le tantale, le niobium, le vanadium et des mélanges de ceux-ci, avec une préférence pour le tantale; C est choisi parmi le magnésium, le calcium, le strontium, le baryum et des mélanges de ceux-ci, avec une préférence pour le magnésium;

x et y sont dans des rapports molaires à z tels que lorsque z=1, alors x=0,001 à 0,25 , et de préférence 0,05 à 0,15 et y=0,001 à 0,25, de préférence 0,002 à 0,20; et

q est le nombre nécessaire pour maintenir l'équilibre des charges avec S, qui est du soufre.

**15.** Procédé suivant la revendication 14 dans lequel ledit composé hydrocarboné aliphatique est du méthane

et ledit composé hydrocarboné aromatique est du toluène.

**16.** Procédé pour la production de chaînes hydrocarbonées aliphatiques et alicycliques insaturées par déshydrogénation, ledit procédé comprenant:

la déshydrogénation d'un composé choisi parmi le groupe comprenant des composés hydrocarbonés aliphatiques, des composés hydrocarbonés alicycliques, des composés hydrocarbonés aromatiques à substitution aliphatique, des composés hydrocarbonés aromatiques à substitution alicyclique, et des mélanges de ceux-ci en présence d'un catalyseur basique constitué de sulfure métallique mixte représenté par la formule xA.yB.zC.qS dans laquelle

A est un métal alcalin choisi parmi le lithium, le sodium,le potassium, le rubidium , le césium et des mélanges de ceux-ci, avec une préférence pour le lithium;

B est choisi dans le groupe comprenant:

un cation qui a un degré d'ionisation une fois plus grand que le degré d'ionisation de C dans lequel B est choisi parmi le scandium, l'yttrium, le lanthane, l'actinium, l'aliminium, le bore et des mélanges de ceux-ci, avec une préférence pour le bore, l'aluminium, l'yttrium et le lanthane lorsque C est choisi parmi le béryllium, le magnésium, le calcium, le strontium, le baryum, le radium, le zinc, le cadmium, le mercure et des mélanges de ceux-ci, avec une préférence pour le magnésium, le calcium, le baryum et le zinc; et B est choisi parmi le titane, le zirconium, le hafnium, le silicium et des mélanges de ceux-ci, lorsque C est choisi parmi le scandium, l'yttrium, le lanthane, l'actinium, l'aluminium, le bore et des mélanges de ceux-ci, avec une préférence pour le bore, l'aluminium l'yttrium et le lanthane; et

un cation qui a un degré d'ionisation deux et trois fois plus grand que le degré d'isonisation de C dans lequel B est choisi parmi le hafnium, le tantale, le niobium, le vanadium et des mélanges de ceux-ci, avec une préférence pour le tantale; C est choisi parmi le magnésium, le calcium, le strontium, le baryum et des mélanges de ceux-ci, avec une préférence pour le magnésium;

x et y sont dans des rapports molaires à z tels que lorsque z=1, alors x=0,001 à 0,25 , et de préférence 0,05 à 0,15 et y=0,001 à 0,25, de préférence 0,002 à 0,20; et

q est le nombre nécessaire pour maintenir l'équilibre des charges avec S, qui est du soufre.

**17.** Procédé suivant la revendication 16 dans lequel ledit composé hydrocarboné est de l'éthylbenzène.